# EUROPEAN PATENT SPECIFICATION

(11) **EP 0 625 267 B1**
(45) Date of publication and mention of the grant of the patent: **11.11.1998**
(21) Application number: 93903248.8
(22) Date of filing: 05.02.1993
(51) Int. Cl.: G01N 33/48, G01N 33/543

(54) **METHODS OF ANALYSIS**
ANALYSEVERFAHREN
METHODES D'ANALYSE

(30) Priority: 08.02.1992 GB 9202705; 14.04.1992 GB 9208235; 09.06.1992 GB 9212178; 13.11.1992 GB 9223795
(43) Date of publication of application: 23.11.1994
(73) Proprietor: GENERA TECHNOLOGIES LIMITED, Birmingham B26 3JZ (GB)
(72) Inventor: PARTON, Adrian, Suffolk CB8 7HX (GB); PETHIG, Ronald, Menia Bridge Gwynedd LL59 5DT (GB); BURT, Julian, Bangor Gwynedd LL57 4LN (GB)
(74) Representative: Smart, Peter John
(86) International application number: GB9300241
(87) International publication number: WO9316383

(56) References cited:
- EP-A- 0 130 530
- EP-A- 0 241 771
- WO-A-90/08961
- DE-A- 3 631 038

## Description

The present invention relates to analytical methods based upon the observation of the rotation of micro-particles in response to a rotating field.

It is known that micro-particles in the form of plastics beads held in suspension in a liquid will rotate in response to a rotating electric field applied through an electrode array. This phenomenon is known as electro-rotation. In a similar manner it is known that a micro-particle presenting a magnetic dipole or in which such a dipole can be induced will rotate in response to a rotating magnetic field. The rotation of a micro-particle in response to the rotation of an applied field, whether electric or magnetic, is referred to herein as "field-rotation".

It has been suggested that electro-rotation could have analytical uses. Thus Arnold and Zimmerman (Natur Wissenschaften, 69 (1982) 297; Z. Natur Forsh., C.37 (1982) 908-915) suggested that a possible application of the electro-rotation of cells could be the detection of interactions between the cell membrane and ecotoxicological substances which affect the electrical characteristics of the membrane such as by altering its permeability to certain ions. Thus it has been found that the electro-rotation properties of a yeast cell may be observably affected by heavy metal ions. This may be due to the electrical characteristics of the cell membrane being changed by the heavy metal ions. Such methods have potential applicability largely only to species which exhibit toxicity to such cells.

The present invention is based upon the observation that the rotational characteristics of a micro-particle can be altered by the formation of a complex involving the micro-particle and some target to be analysed and that by observation of the distinct rotational properties of the complex, information regarding the target can be derived.

Whilst methods according to the invention may be employed in a wide variety of analytical applications, they have particular relevance to the analysis of samples containing micro-organisms or micro-biological products. Current methods of identifying micro-organisms themselves, e.g. bacteria, involve the use of immunological, DNA, biochemical and micro-biological techniques. Traditionally, standard micro-biological techniques such as plating the organisms out and observing their growth on selective media have been employed for the detection of specific organisms. More recently, techniques such as immunoassay involving the use of antibodies directed at the specific organisms in question and methods of amplifying and detecting characteristic DNA sequences have become widely used. Methods such as immunoassay and DNA detection provide advantages in that they have some specificity and are invariably faster than using more traditional biological techniques. However, the past techniques suffer from limitations amongst which are that they are restricted in their degree of sensitivity, that in some cases they require very skilled operatives and laboratory resources, that they tend in their nature not to be very quantitative and that only the traditional plating techniques discriminate between viable and non-viable cells.

Methods described hereafter according to the present invention allow some or all of these drawbacks to be addressed.

The present invention provides a method of analysis comprising forming a complex between a micro-particle, a linking moiety attached to the micro-particle and a target, which complex has field-rotation properties observably distinct from those of said micro-particle and attached linking moiety alone, and observing the said distinct field-rotation properties of the complex.

The "target" referred to above need not itself be the species to establish the presence, nature or quantity of which is the ultimate purpose of the analysis. Thus, the target may be a reagent in the analysis and the species of interest in the analysis may be another component of the complex, e.g. the linking moiety.

By way of background, when a micro-particle is exposed to an external electric field, electrical charges are induced to appear on the surfaces of the particle so that it acquires an electrical dipole moment. If the electric field is rotated, a torque acts on the particle of a magnitude proportional to the induced dipole moment and the field magnitude so as to cause the particle to spin. Methodologies for generating rotating electric fields and for the visual observation of the resulting electro-rotation of colloidal particles, including biological cells, have been described by W.M. Arnold and N. Zimmerman (Journal of Electrostatics, vol. 21, pp.151-191 (1988) and R. Holzel (Medical and Biological Engineering and Computing, vol. 28, pp.102-105 (1988)). The sense of rotation of the particle depends on the dielectric and electrical conductive properties of the particle and the . surrounding suspending medium and may either be in the same sense as that of the rotating electric field (known as co-field rotation) or in the opposite sense (known as anti-field rotation). The rate of particle electro-rotation is typically of the order of 2 to 6 radians per second which is of course sufficiently slow that it can be observed directly through a microscope and the rotation rate of the particles can be measured simply by an observer counting the number of rotations within a period of time. The phenomenon is dependent on the frequency of rotation of the applied electric field and depending upon the nature of the micro-particle, field rotation rates of from 10 Hz to 100 MHz can be used.

The torque experienced by the micro-particle in the rotating electric field may be viewed as being the consequence of a lag between the rotation of the field and the rotation of the induced dipole of the micro-particle. The dipole induced in the micro-particle can be realigned with the field without rotation of the particle through the decay of the dipole induced in a first field orientation and the formation of a new dipole aligned with a new field orientation. Insofar as this process of decay and repolarisation lags behind the applied rotating field, the effective dipole moment of the micro-particle is out of alignment with the field at any given time and hence a torque is applied to the particle. The dependence of the observed electro-rotation phenomenon on the rotation frequency of the applied field is accordingly understandable in the following terms. For very low excitation frequencies, the rearrangement of the dipole in the particle can keep pace with the rotating field and the lag between the dipole and the field is very small so that rotation is not provoked. Within a range of frequencies, the dipole lags behind the field and electro-rotation is observed. At very high frequencies, the dipole cannot follow the field sufficiently quickly to maintain any constant relationship to it and no dipole is induced so that there is no electro-rotation torque.

We have found that the electro-rotation properties, e.g. the rate of particle rotation observed at a given field rotation frequency, for a given micro-particle can be altered sufficiently by the formation of a complex between the micro-particle and a target species to be analysed such as a micro-organism cell that the change in electro-rotation properties can be observed and can form the basis of an analytical method. As will be shown hereafter, within this broad concept there are methods of analysis capable of distinguishing between viable and non-viable micro-organism cells and methods of analysis capable of a degree of sensitivity not previously obtainable. If desired, quantitative information can be obtained.

Preferably therefore, the field rotation properties observed are electro-rotation properties.

The linking moiety bound to the micro-particle is a species having an affinity for the target. Preferably, the said affinity for the target is a selective affinity such that the formation of the complex between the micro-particle and the target is selective and provides at least a degree of identification of the target. Preferably, said affinity is highly specific and accordingly the said linking moiety bound to the micro-particle substrate which provides the selective affinity for the target may be an antibody or an antibody fragment having antibody activity, an antigen, a nucleic acid probe or a nucleic acid analogue probe having selective affinity for complementary nucleic acid sequences, or avidin or an avidin like molecule such as strept-avidin.

Antibodies and antibody fragments having antibody properties are particularly preferred. There are known techniques suitable for coating antibodies on to the surface of particles such as plastics micro-beads which are well known to those skilled in the art. Antibody coated micro-particles are capable of recognising and binding corresponding antigens which may be presented on micro-organism cells or some other target.

Methods are also known for binding oligo-nucleic acid probes to such micro-particles. Suitable techniques are by way of example described in PCT Application No. GB92/01526. Where the linking moiety is a nucleic acid probe or a nucleic acid analogue probe, the micro-particle will of course be suitable for recognising and binding complementary nucleic acid sequences.

The said complex further may comprise a label bound to said target. The label may be bound to said target either before, simultaneously with, or after the formation of the complex between the target and the micro-particle. The label may comprise a second linking moiety having an affinity for said target and a labelling moiety carried by said linking moiety. Once again, it is preferred that the affinity for the target possessed by the second linking moiety is selective, preferably highly specific and the second linking moiety may also be an antibody, an antibody fragment having antibody activity, and antigen, a nucleic acid probe, a nucleic acid analogue probe, avidin or an avidin like molecule. The use of a label of this nature may be desired where a complex between the micro-particle and the target does not in itself possess sufficiently distinctive field rotation properties. The field rotation properties may be further altered by the inclusion in the complex of the label. To this end, the labelling moiety carried by the linking moiety in the label may be a micro-organism, a metal particle, a polymer bead or a magnetic particle. For use in connection with electro-rotation measurements, the label preferably has dielectric properties and is capable of acquiring a significant surface charge. A particularly preferred material is colloidal gold which is easily bound to antibodies (as said second species) to form a label. Antibodies bound to colloidal gold are commercially available and methods for binding antibodies to colloidal gold are for instance described in Geohegan W.D. et al (1978) Immunol. Comm 7 pl. Other metal particles however may be employed, e.g. silver particles and iron particles.

The use of a label of the kind described above may be preferred even where a complex between the target and a micro-particle possesses sufficiently distinctive electro-rotation properties to enable the formation of such a complex to be observed. A higher level of specificity may in certain cases by obtained by the use of a label in such a complex. Thus for instance, a micro-organism expressing both a first antigen and a second antigen might be distinguished from a micro-organism expressing only the first antigen by the use of micro-particles having as a linking moiety an antibody to the first antigen and a label having as its second linking moiety an antibody to the second antigen, the distinct field rotation properties of the complex between the micro-particle, the micro-organism and the label being observed and distinguished from those of the micro-particle and the micro-organism expressing only the first antigen.

The label may include a magnetic particle so that the label can be attracted to a magnet so as to concentrate Complexes containing said label for easier observations. In some cases it may be possible to attract labelled complexes to a magnet and to wash away unlabelled beads so as to eliminate the background of beads bearing linking moieties but no target/label which would normally be present. Suitable magnetic labels for this purpose will include iron micro-particles bearing linking moieties such as antibodies. Such antibody coated in iron particles are commercially available.

Usually it will be preferred to employ an electric field rotating at from 10 Hz to 1 MHz, more preferably from 100 Hz to 1000 Hz e.g. about 300 Hz.

Preferably the electro-rotation properties are observed using a rotating electrical field having a strength of from 50 to 650 volts per centimetre preferably, 100 to 500 volts per centimetre, e.g. about 200 V/cm.

A generally preferred form of electro-rotation apparatus takes the form of a sample receiving surface having a set of electrodes arranged thereon surrounding a sample analysis field in which electro-rotation is to be observed, a light source directed at the said field and means for observing particles in the field, such as a microscope or a camera. The apparatus will typically further include a signal generator for applying a chosen voltage in the electrodes at a chosen frequency to produce a rotating field.

Typically, one may employ an analysis field between the electrode of approximately 0.01 to 0.3 mm², e.g. 0.08 mm² area, by using electrodes printed on the sample receiving surface and approaching one another to within 100 µm to 0.6 mm. Four electrodes having arrow shaped ends approaching a common point from four orthogonal directions may be used. A quadrature output from a signal generator may be applied to them.

However, larger analysis fields may be used, particularly if the configuration (number and spacing) of the electrodes is suitably chosen and larger voltages are employed.

The field rotation properties of the complex may be observed directly through a microscope, the observer measuring the rotation rate of individual particles within the microscope field in turn and optionally noting the number of particles which are observable.

More preferably however, the observation of the field rotation properties is conducted on an automated basis using optical detection techniques, e.g. image analysis techniques which may be used to detect and characterise the individual rotation profiles of several or all complexes within the field of view of the apparatus.

The micro-particle substrates employed are preferably polymer beads, more preferably polystyrene beads. The polymer beads may be made magnetic as an aid to their handling in processing steps or to assist their visualisation in electro-rotation measurements as described hereafter.

The beads may be spherical but it may be preferable to employ beads which have visually distinguishing features such as surface pits or marks or asphericities to assist in visualising the rotation of the beads.

As it is difficult to observe the rotation of spherical beads using image analysis due to the lack of features which can be observed to track the beads' rotation, beads can optionally be marked to provide features to track.

Preferred marking methods include coating beads or other microparticles with a dye, e.g. a fluorescent dye, and then further treating the dye-coated beads to make the dye coat visually patchy. This may for instance be a physical treatment such as using a laser to alter the dye, e.g. bleach it. One side of the beads may be illuminated by a laser to produce this effect so that when illuminated under UV light, the beads have a fluorescent hemisphere and a dark hemisphere so that rotation is easily observed.

A dye, e.g. a fluorescent dye, may be incorporated as a label into the linking moiety to be attached to the beads or other microparticles. Once again visually observable patches can be produced by laser illumination.

In either of the above methods a chemical treatment may be used instead of the laser treatment to produce the required patchy dye coating.

Alternatively, dye may be applied to the beads in a nonuniform way to produce the visual patchiness directly. Thus one may use dye diffusion so transfer dyes to only one side of the mass of beads or other microparticles.

A further suitable approach is to provide a first coating on a first set of microparticles and a second coating on a second set of microparticles, the first and second coatings having an affinity, e.g. a selective affinity, for one another. Antibody/antigen coatings may be used. The beads when mixed will bind to each other to form composite microparticles which are easily observable.

Preferably, one set out of the first and second sets of microparticles is constituted by particles of a diameter small compared to that of the microparticles in the other set. The composites then take the form of a larger microparticle surrounded by smaller ones. The linking moiety for the assay may be coated into the smaller particles. Suitable particle diameter ratios are from 1:4 to 1:8, e.g. ≤1µm particles and 6µm particles.

The invention includes a kit for use in analysis by electro-rotation comprising micro-particles bearing linking moieties adapted to form a complex with a particular target species and at least one of:-
(a) a sample receiving surface having thereon a pattern of electrodes surrounding an analysis field suitable for use in an electro-rotation assay; or (b) an electro-rotation label adapted to form a complex with the same target species.

The sample receiving surface may be provided on a permeable member capable of filtering a sample to concentrate said target species. For instance, to concentrate micro-organisms, the electrodes may be on the surface of a micro-porous filter, e.g. a membrane through which a volume of sample is passed to trap micro-organisms on the membrane for binding to the micro-particles. The kit may then include means for passing a volume of liquid through a permeable member providing said sample receiving surface and preferably means for measuring said volume.

In a separate aspect of the invention includes for use in an electro-rotation assay a micro-porous filter member bearing a pattern of electrodes defining a sample receiving field and capable of applying a rotating electrical field to a sample thereon.

The broad range of analytical techniques made available according to the invention will be illustrated by the following discussion of a number of general examples of methods of analysis within the scope of the invention.

Reference will be made to the accompanying drawings in which:-
Figure 1 is a schematic side elevation of measurement apparatus according to the invention;
Figure 2 is a schematic illustration of the electrode arrangement of electro-rotation measurement apparatus shown in plan view;
Figure 3 is a schematic side elevation of a further form of measurement apparatus for use in the invention;
Figure 4 is a schematic illustration of the complex between a micro-particle and a bacterium cell;
Figure 5 is a schematic illustration of a complex between a micro-particle, a target and a label;
Figure 6 illustrates the detection of a nucleic acid target using a capture probe attached to a polymer bead and a detection probe bearing a labelling moiety;
Figure 7 illustrates the detection of a nucleic acid amplification product incorporating an electro-rotation label originally present in a primer;
Figure 8 shows a modification of what is shown in Figure 7 where the target contains a first label detectable by reaction with a suitable electro-rotation label;
Figure 9 shows a similar arrangement to Figure 8 using biotin as a label incorporated into the target by using biotinylated nucleotides in an amplification reaction; and
Figure 10 illustrates the pclymerase mediated extension of a probe primer carried by a polymer bead to form an extended linker moiety which can capture an electro-rotation label acting as the required target, the presence of the extended linker moiety being the actual subject matter of the analysis.
Figures 11 to 16 show the results of electro-rotation analyses described in detail in Examples 1 to 8. The Figures and Examples relate to one another as follows:-

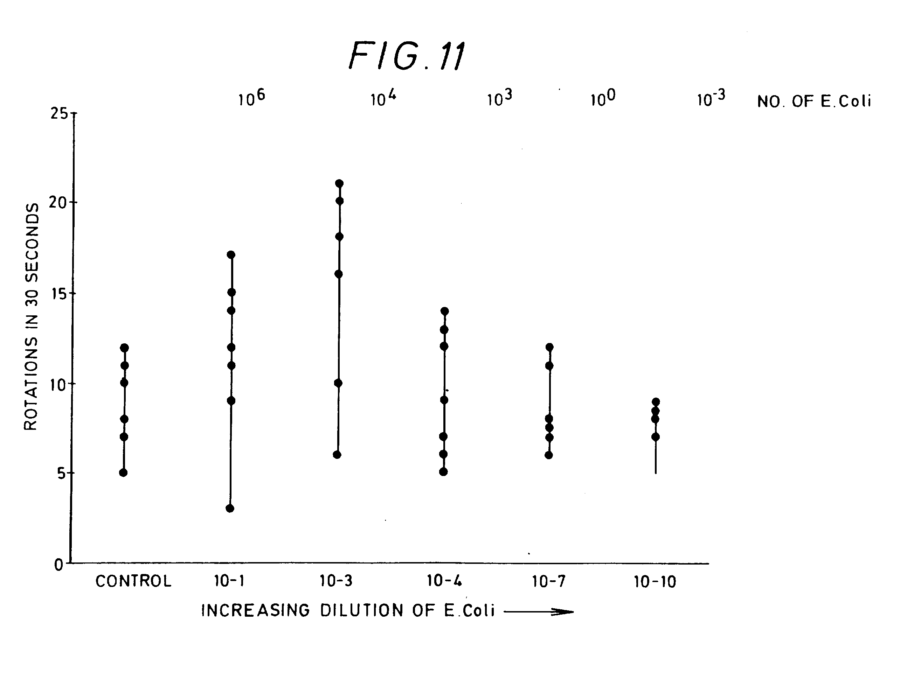

   Figure 11 - Example 3

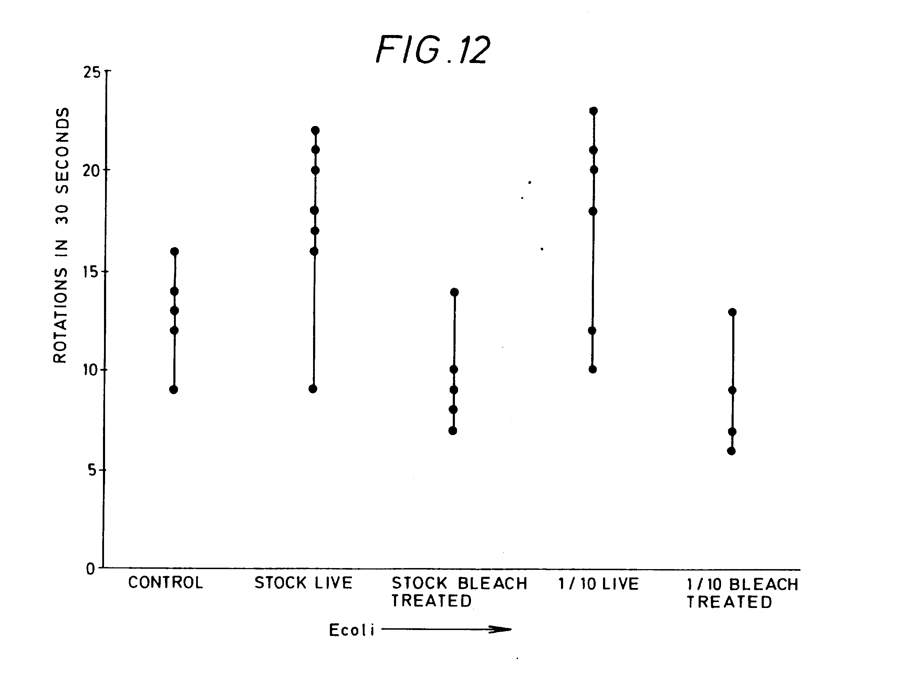

   Figure 12 - Example 4

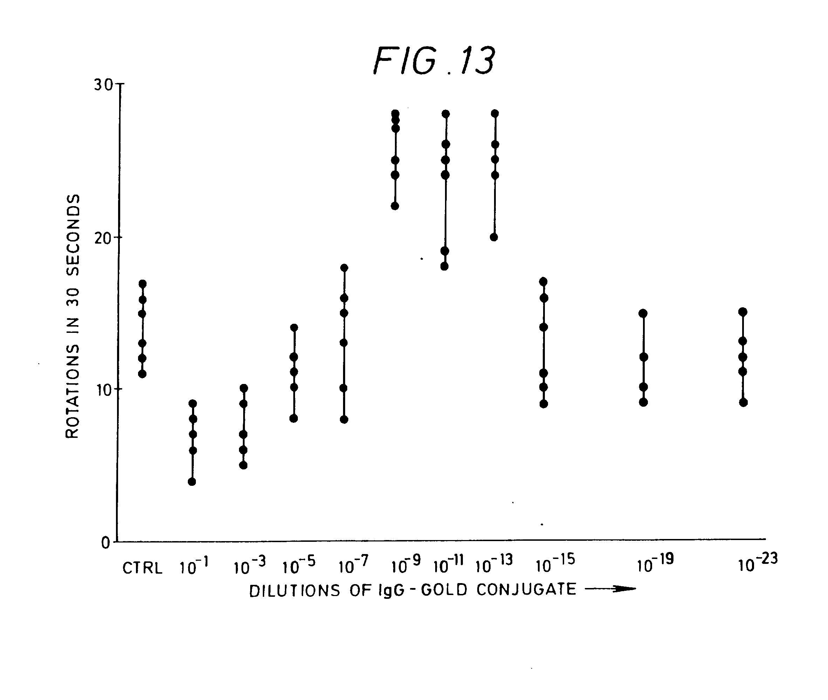

   Figure 13 - Example 5

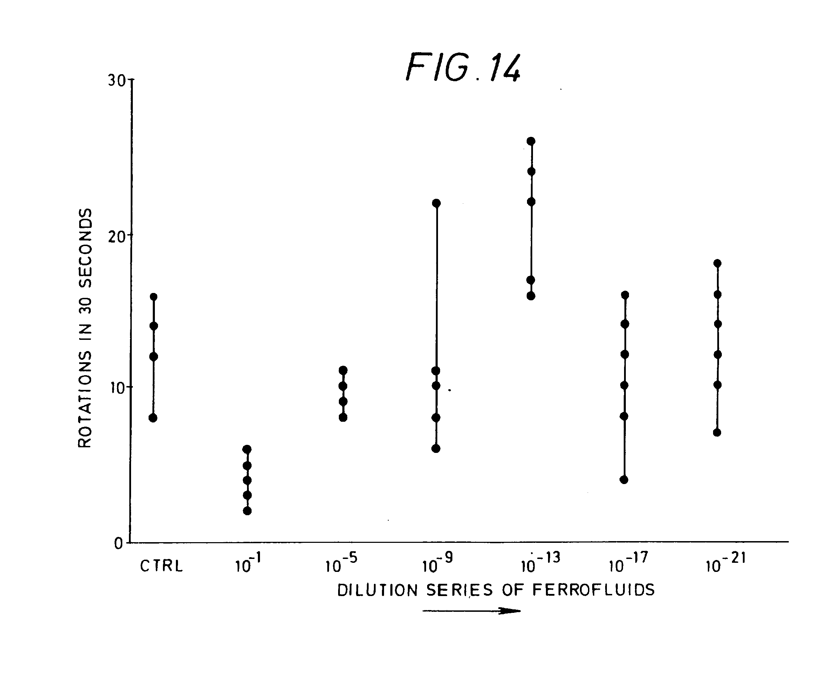

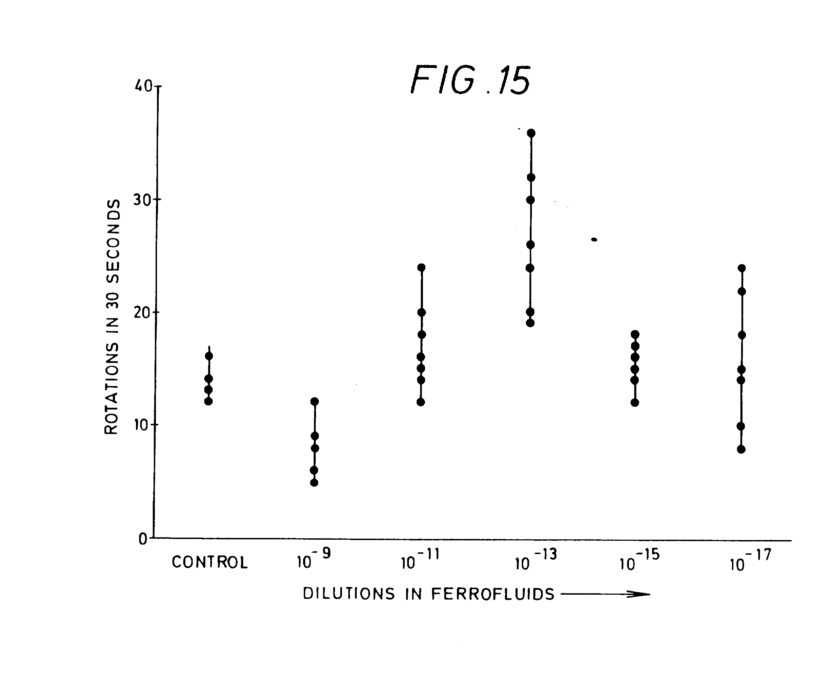

   Figures 14 and 15 - Example 6

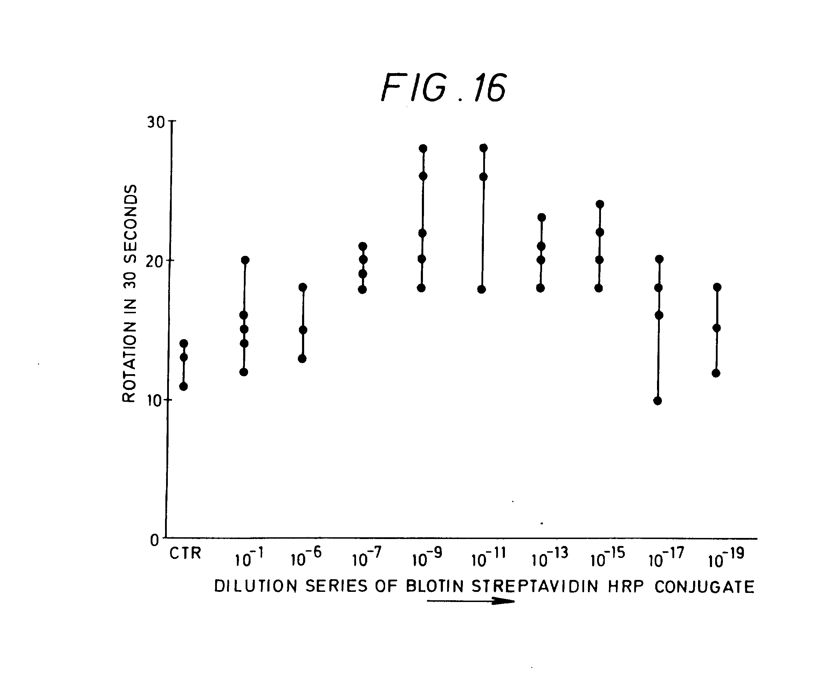

   Figure 16 - Example 7

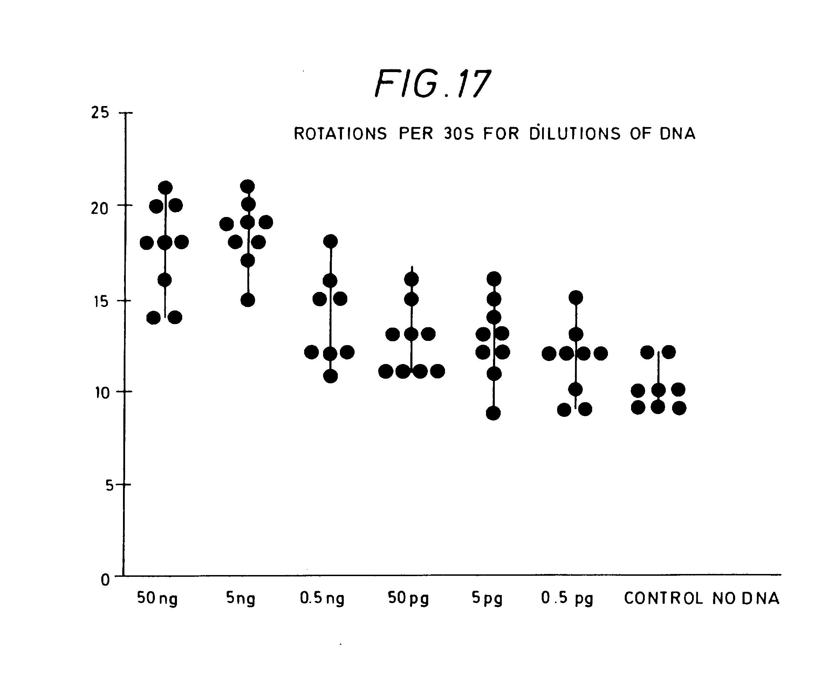

   Figure 17 - Example 8.

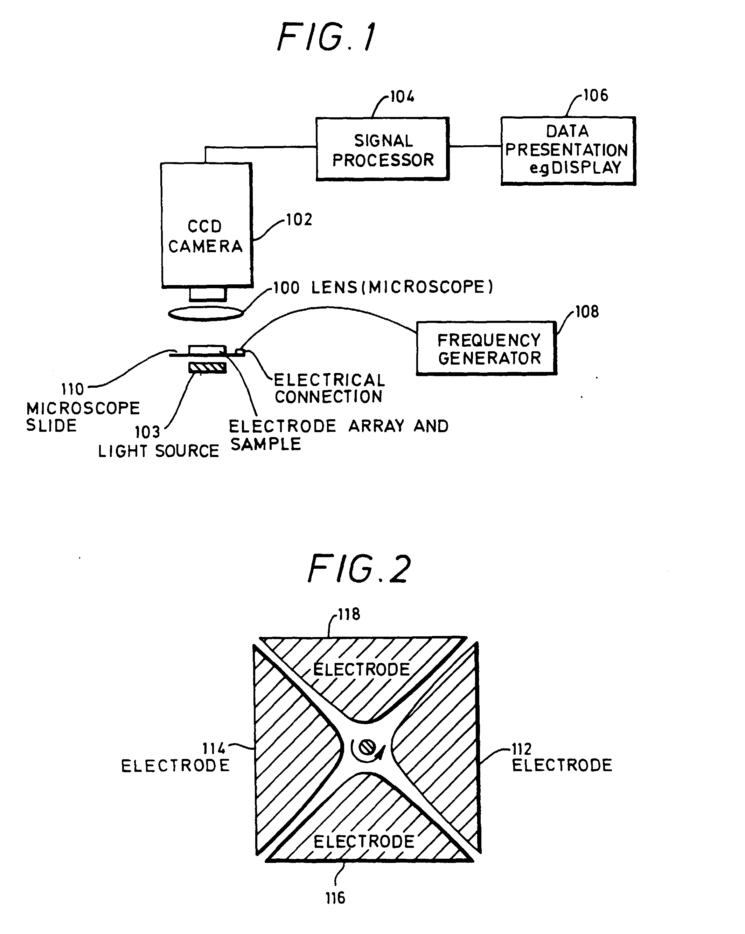

As shown in Figure 1, apparatus for use in accordance with the invention comprises a light microscope schematically indicated by a lens 100 which is equipped with a CCD camera attachment 102 as well as a conventional field illuminating light source 103 and slide carrier stage (not shown).

The CCD camera 102 is connected to a signal processor unit 104 and to a display screen 106. The apparatus further comprises a frequency generator 108 which in use is connected to terminals provided on a microscope slide 110 which bears a pattern of electrodes 112-118 as shown in more detail in Figure 2.

The electrodes may be printed, e.g. in gold, or coated and etched on the microscope slide 110 together with tracks leading to terminals for connection to the frequency generator 108. The spacing between opposite electrodes 112 and 114 and between opposite electrodes 116 and 118 is preferably in the region of 200 to 500 µm. In the apparatus used to produce the results described with reference to the examples below, the spacing was 315 µm. The frequency generator provides two sine wave outputs 90° out of phase ( a quadrature output), one of which is connected between electrodes 112, 114 and the other of which is connected between electrodes 116, 118. If desired, a greater number of electrodes may be employed which may provide a more uniform rotating field. The frequency generator is provided with variable outputs to enable the operator to set the frequency of the sine wave outputs and the peak to peak voltage of them. Typical conditions employed in the examples which follow were 300 Hz at 6 volts peak to peak.

In use, the image appearing on the display of the apparatus shows a number of beads within the field of the microscope rotating sufficiently slowly that the number of rotations within a period such as 30 seconds can be counted directly by an observer looking at each bead in turn. Whilst apparatus as described above is sufficient to produce useful results as demonstrated by the specific examples given below, it is generally preferable that rotation speed measurements be carried out on more beads than a human observer could reasonably deal with directly. For this purpose, it will be desirable to use image processing techniques to detect and measure the rotation of the beads in the apparatus. From the CCD camera attachment, one may derive images of a field of view containing a number of rotating beads. A series of such images, or frames, may be captured by frame-grabber circuitry in a computer and the series of images may be analysed by image processing software.

Thus, a thresholding process may be used to convert the grey scale image produced by the camera into a binary image. A separate threshold is performed to identify any areas of the image which are considered too dark, these areas being removed from the binary image of the beads. The remaining shapes in the image may be separated from the rest of the image and subjected to a test of one of more characteristic properties, such as area, to select those which are likely to represent beads. The centre of mass and orientation may then be determined for each qualifying shape. The positions of the centres may be compared between the successive frames in order that each bead in one frame may be located in the next. Once a bead has been located in two frames, the difference in the orientation of the bead will give directly the amount through which it has rotated from which its rotational speed may be calculated and averaged over several pairs of frames. By such automated techniques, the rotational speeds of all of the beads within the field of view at any moment may be measured and a statistical picture of the rotational characteristics of the beads may be developed, optionally using a number of field rotation frequencies.

The apparatus may be further modified by the inclusion of a magnet, suitably an electro-magnet, positioned to draw any magnetic beads in the sample into the field of view of the microscope. The magnet would then be removed or turned off whilst the rotational characteristics of the beads were measured.

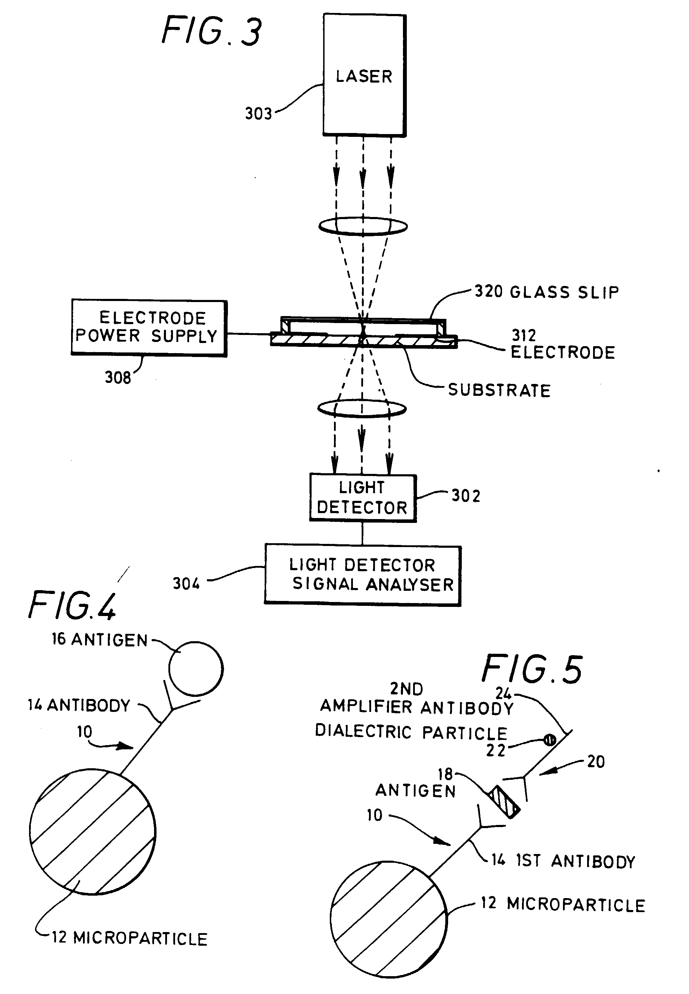

It is not essential that a visual image capable of human interpretation is produced by the apparatus. As shown in Figure 3, apparatus for use in the invention may include a laser light source 303 illuminating a sample slide through suitable optics to produce an image on light detection apparatus 302 which is fed to signal analysis circuitry 304 to provide a numeric output of the rotational characteristics observed when a rotating electric field is applied to the sample slide via electrodes 312 from a frequency generator 308. The slide bears a coverslip 320.

As shown in Figure 4 a micro-particle may comprise a plastics bead 12 having bound to it as a linking moiety one or more antibody molecules 14. It should be noted that the drawing is not to scale and that normally in reality the plastics bead will be coated with a multitude of antibody molecules. The micro-particles may be exposed to micro-organism cells 16 bearing surface antigens reactive with the bound antibody to form a complex 10 between the micro-particle and the cell. The micro-organism cells may for instance be E. coli or coliforms present in water or pathogenic micro-organisms present in foodstuffs such as listeria or salmonella. It has been found that the rate of rotation of such micro-particle/micro-organism cell complexes under suitably chosen conditions is distinguishable from the rotation rates obtained using the micro-particles alone and furthermore that the rotation rates obtained for complexes between micro-particles and viable micro-organism cells can be distinguished from those obtained between micro-particles and similar non-viable cells.

As shown in Figure 5, a ternary complex 10 may be produced between a micro-particle 12 of the kind shown in Figure 4 as described above, an antigen 18 and a label 20 comprising a labelling moiety 22 and a second antibody 24 which may be the same as or different from the antibody 14. A ternary complex of this type may be employed where the antigen 18 is too small or lacking in dielectric properties sufficiently to affect the electro-rotation properties of the micro-particle 10 or where it is desired to obtain a higher level of specificity through the use of two different antibodies in a single analysis. The antigen may for instance be a toxin present as a contaminant in a foodstuff.

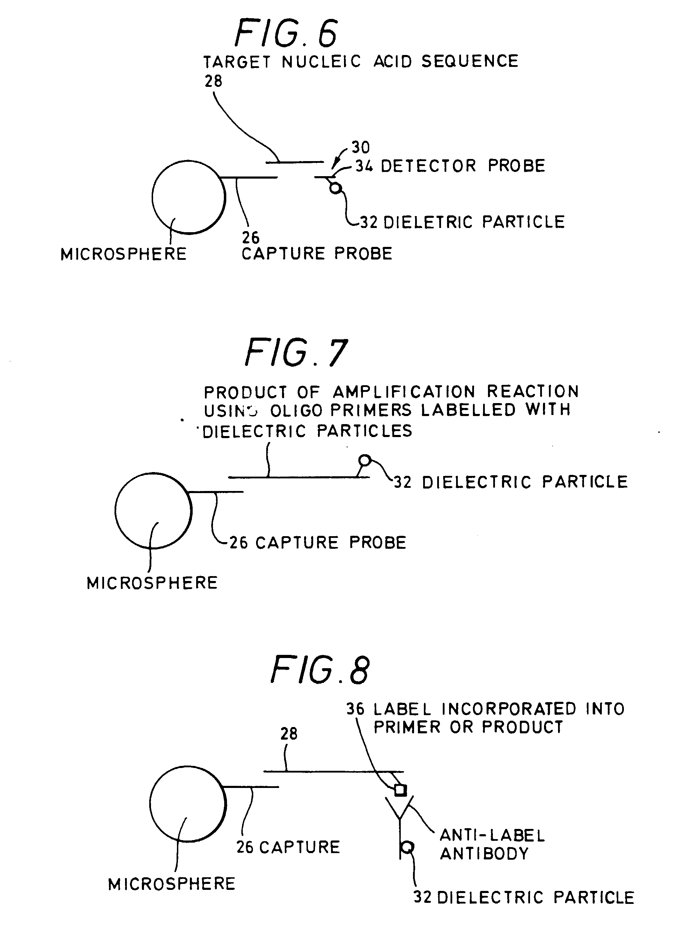

Figures 6 to 11 illustrate methods of detecting nucleic acids and in particular nucleic acid sequences produced as products of amplification procedures such as PCR, LCR and 3SR techniques. Known methods of detecting nucleic acid amplification procedure products rely upon them undergoing some form of purification and/or separation procedure before they are analysed. One commonly used method involves the analysis of the products by agarose gel electrophoresis. This separates the amplified DNA fragments and any remaining oligonucleotide primers on the basis of size. Separating products on the basis of size alone does not enable one to distinguish between PCR products of the required base sequence and an amplified contaminant of approximately the same length. In order to further identify PCR products, the gel electrophoresis may be taken one step further by subjecting its product to the Southern blotting technique in which the separated fragments are transferred from the gel to a membrane in direct correspondence to their relative positions on the gel. They are then probed with a labelled single stranded DNA probe with a base sequence complementary to the sequence of interest. The label used for the probe is usually biotin or a radio-isotope such as ³²P. Southern blotting is relatively laborious and requires a moderate level of laboratory skill as well as laboratory equipment and facilities. It is not suited for use in rapid screening of a large number of samples or the screening of samples outside the laboratory. The method of dot blotting in which the agarose gel separation step of Southern blotting is omitted is somewhat quicker but basically. suffers from the same disadvantages as Southern blotting.

As illustrated in Figure 6, in a method according to the present invention a microsphere may include an oligonucleotide or synthetic oligonucleotide analogue as a capture probe 26 (linker moiety) bound to the surface of a polymer bead and having a sequence complementary to that of an expected amplification procedure product 28. A label 30 comprising an electro-rotation labelling moiety 32 as described above bound to a second oligonucleotide or oligonucleotide analogue sequence 34 complementary to a second region of the target nucleic acid sequence is employed. The micro-particles and the label may be added to the product of the amplification reaction before or after any working up of the reaction mixture to separate the amplification products. The electro-rotation properties of the micro-particle/amplification product/label ternary complex may then be observed and distinguished from those of the micro-particles alone. A variant on this procedure is illustrated in Figure 7 in which one of the primers used in the amplification procedure is labelled with a suitable electro-rotation labelling moiety 32 which thereby becomes covalently incorporated into the nucleic acid product of the amplification procedure. A binary complex is then produced between a micro-particle bearing a capture probe 26 and the amplification product and the electro-rotation properties of the complex are observed.

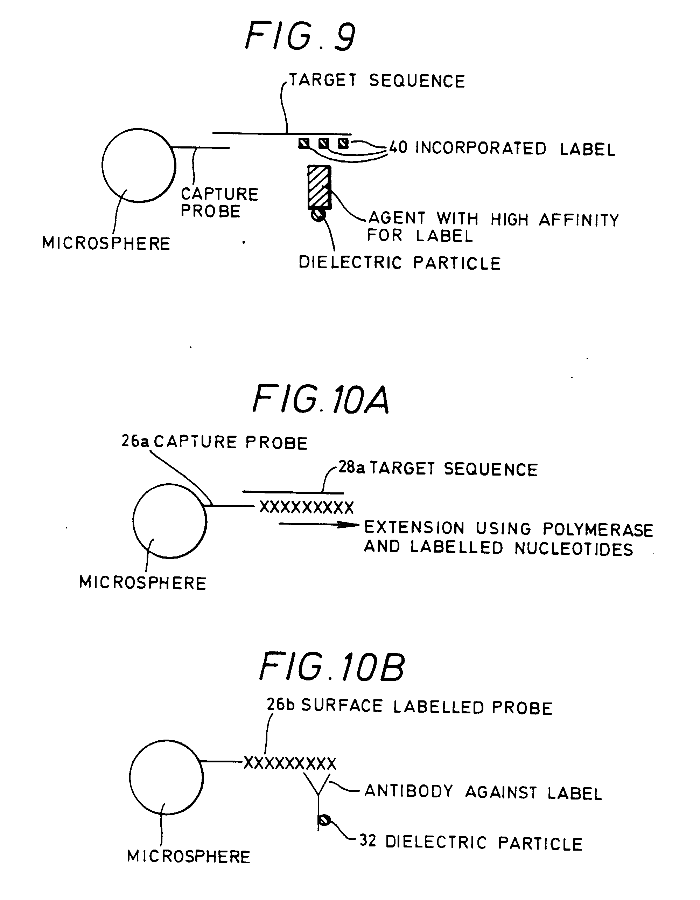

A further variant is shown in Figure 8 in which the label incorporated into the primer in the amplification procedure is not itself an electro-rotation labelling moiety but is instead a label 36 chosen to have an affinity with the second linker moiety of an electro-rotation label as described above which itself incorporates an electro-rotation labelling moiety 32. The label 36 may for instance be reactive with an antibody bound to the labelling moiety 32 or may be biotin label reactive with an avidin or avidin like molecule bound to the labelling moiety 32. In a variation on this procedure, an antibqdy bound to the labelling moiety 32 may be one raised against any nucleic acid sequence present in the amplification product. In a variant on this procedure shown in Figure 9, rather than incorporating the label into the primer, the label may be incorporated into some or all of the nucleotides used in the amplification procedure, e.g. one may employ biotinylated nucleotides 40. A label may then be employed comprising an electro-rotation labelling moiety bound to avidin or an avidin like molecule such as streptavidin.

In the method illustrated in Figures 10a and 10b, a target sequence 28a is first hybridised to a capture probe 26a attached to a micro-particle substrate and the amplification procedure is continued or a fresh amplification procedure is commenced to extend the capture probe of the micro-particle through the use of polymerase and labelled nucleotides, e.g. biotinylated nucleotides. The target sequence may then be dehybridised from the micro-particles leaving the labelled extended probe 26b bound to the micro-particle substrate. The label on the probe may then be reacted with an electro-rotation label comprising an electro-rotation labelling moiety 32 and a species reactive with the labelled probe, e.g. avidin. One thereby forms a complex between the micro-particle bearing as a linking moiety the extended capture probe 26b and the label incorporating the labelling moiety 32 acting as a target for an electro-rotation assay.

Alternatively, the extended capture probe need not incorporate any label. Instead the labelling moiety 32 may be attached to an oligonucleotide on nucleic acid analogues complementary to the extended capture probe.

In certain cases, it may even be possible to observe the change in electro-rotation characteristics brought about by the extension of the capture probe without using any further label. In such a case, the extension of the capture probe constitutes the target species with which the complex with the micro-particle (bead) and linking moiety (capture probe) may be said to be formed.

It will be appreciated that the nucleic acid entity which is detected according to the methods exemplified with reference to Figures 6 to 10, particularly those illustrated in Figures 6 and 10, need not necessarily be the product of an amplification procedure.

The invention will be further illustrated by the following specific examples:-

### Example 1

### Preparation of Antibody Coated Polymer Beads

Polystyrene micro-particles of 6 µm diameter (Polysciences Limited) were employed as a stock suspension containing 2.1 x 10⁸ beads per ml. 500 µl of the bead stock was centrifuged at 6,000 rpm in an Eppendorf centrifuge for 1 minute. The supernatant was discarded and the beads were resuspended in 1 ml PBS (phosphate buffered saline). The process was repeated three times to wash the beads. The final resuspension was in PBS containing antibody at 100 µg ml⁻¹ or 300 µg ml⁻¹. The beads were placed in a tube tumbler for passive binding of the antibody to occur overnight at room temperature. Thereafter the suspension was spun and the supernatant was discarded. The beads were resuspended in 1 ml of 1% bovine serum albumin in PBS. After tumbling for 30 minutes at room temperature, the beads were washed into PBS by the procedure described above.

The resulting antibody coated beads each have a large number of antibody molecules coated on their surface.

### Example 2

### Formation of Antibody Coated Bead - E. coli complexes

A stock solution containing approximately 10⁷ E. coli organisms per 100 µl was employed, the concentration of organisms present being checked by standard techniques.

Dilutions of the stock solution were made by factors 10⁻¹, 10⁻³, 10⁻⁴, 10⁻⁷ and 10⁻¹⁰.

The stock solution and each dilution were separately complexed with antibody coated beads prepared as in Example 1 using antibody supplied by Biogenesis Ltd. The antibody coated beads were spun out of suspension and resuspended in the suspension of E. coli in PBS each time at a bead concentration of 300 micrograms per ml. Binding of the bacteria to the beads was allowed to continue for 1 hour at 37°C prior to rotation measurement.

### Example 3

### Electro-rotation of Antibody Coated Polystyrene Bead - E. coli Complexes

A 15 µl sample of each suspension of complexes prepared in Example 2 was placed on the centre of the electrode array of the slide described above with reference to Figure 2 and covered by a coverslip. The slide was placed on the microscope platform of the apparatus described with reference to Figure 1 and the beads was electro-rotated using a 6 v peak to peak 300 Hz field. The rotating beads were visualised using the CCD camera connected to a television screen and the number of rotations performed by individual beads in 30 seconds was measured by eye and stop watch. The results are shown in Figure 11. Each point in the figure represents the rotation rate observed for a particular individual particle under the microscope. It can be seen that a spread of rotation rates was observed for control beads (antibody coated beads incubated in the absence of E. coli) but that substantially faster rotating beads could be seen at both 10⁻¹ and 10⁻³ dilutions. The 10⁻³ dilution corresponds to approximately 10,000 E. coli organisms in the whole 100 µl sample employed. It may be observed that the rotation rate seen at the 10⁻¹ dilution is somewhat less than at the 10⁻³ dilution. This may be because more E. coli organisms are then binding to each bead observed. This would produce a complex which is intrinsically less asymmetric in its dielectric characteristics than if the complex consists of a single bead and a single organism.

### Example 4

### Comparison of Electro-Rotation Properties of Live and Non-Viable E. coli

A suspension of non-viable E. coli organisms was produced by treating the stock suspension of live E. coli (10⁷ organisms per 100 µl) referred to above with bleach and the non-viability of the organisms after bleach treatment was checked by plating out on a suitable growth medium. The electro-rotation characteristics of the stock live suspension and the stock bleach treated suspension were compared after the formation of antibody coated bead - E. coil organism complexes as described above in Example 3 and this procedure was carried out also using 10⁻¹ dilutions of the live and bleach treated stock suspensions. The results are shown in Figure 12. It can be seen that whilst the non-viable bleach treated organisms produce electro-rotation at rates marginally slower than control, the live organisms produce electro-rotation at significantly higher speeds thus demonstrating the ability of the electro-rotation assay to distinguish between viable and non-viable E. coli at dilutions at least down to 10⁶ organisms per 100 µl of sample.

### Example 5

### Electro-Rotation Analysis of Antibody Coated Polystyrene Bead - IgG Gold Conjugate

Commercially available gold particles complexed with approximately 4 IgG antibody molecules per gold particle (10 nm diameter) were used to form complexes with anti-IgG antibody coated beads prepared as described in Example 1. A stock suspension of IgG-gold conjugate particles containing 10¹³ to 10¹⁵ gold particles per 100 µl was used together with increasing dilutions ranging between 10⁻¹ and 10⁻²³ fold and the resulting complexes were subjected to electro-rotation analysis as described above in Example 3. The results are shown in Figure 13. The control represents antibody coated beads not exposed to the IgG gold conjugate. It can be seen that the presence of the IgG-gold conjugate can be determined down to dilutions of 10⁻¹³ of the stock suspension at which level it is probable one is observing the rotation of beads to which only a single IgG-gold particle has complexed.

### Example 6

### Electro-Rotation Analysis of a Ferrofluid (Iron particle - Antibody Complex)

The previous example was repeated using in place of IgG-gold complexes particles consisting of iron complexed with numerous molecules of antibody (diameter approximately 16 nms). Once again dilutions of a stock solution containing approximately 10¹³ iron particles per 100 µl were used ranging from 10⁻¹ down to 10⁻²¹ fold. The results for different dilutions are shown in Figures 14 and 15.

### Example 7

### Electro-Rotation Assay of Biotin using Polystyrene Bead - Antibiotin complexes and Streptavidin - Horseradish Peroxidase Conjugate

A stock solution and a range of dilutions thereof containing approximately 2.4 x 10¹⁴ biotin molecules per 100 µl (stock concentration) were incubated with suspensions of antibiotin coated polystyrene micro-beads and streptavidin horseradish peroxidase (HRP) conjugate to produce electro-rotation complexes consisting of a micro-particle bound via antibody to biotin to which is also bound horseradish peroxidase as an electro-rotation label. The results for a dilution series ranging from 10⁻¹ to 10⁻¹⁹ fold dilution of the stock solution are shown in Figure 16. Raised rotation rates are observable at least down to 10⁻¹⁷ fold dilutions which correspond to only about 2,400 biotin molecules per 100 µl.

### Example 8

### Electro-Rotation Analysis of Polystyrene Bead - Oligonucleotide Capture Probe - PCR Product complexes

### Attachment of Capture Probe to Beads

A 39 bp long capture probe, recognising 20 bp from the coding sequence from the hlyA gene in Listeria Monocytogene (the complement of the sequence from base 858 to 877 according to Mengaud et al., 1986) was synthesised. The oligonucleotide was then 5-prime phosphorylated according to Maniatis et al (1991); by incubation with T4 polynucleotide kinase in the presence of ATP and commercially supplied buffer for 2 hrs at 37°C.

The phosphorylated oligonucleotide was attached to polystyrene microspheres with surface amino groups (Polybead Amino Microspheres, 6µm diameter Polysciences Inc.) by incubating overnight in 0.1 M Imidazol buffer pH7 and 0.1 M 1-ethyl-3-(3-dimethylaminopropyl)-carbodiimide (Lund et al., 1988).

### Capture Reaction

Prior to the capture reaction the target DNA (144 bp double stranded biotin labelled DNA fragment produced by PCR, using primers flanking bases 757 to 901 in the hlyA gene, Mengaud et al., 1986) was denatured for 5 minutes at 95°C in a range of concentrations per 100 µg of sample as shown in Figure 17. The reaction contained target DNA, polystyrene beads with capture probes, and PCR buffer. Annealing of the target sequence with the capture probe took place at 55°C for 15 minutes, followed by a washing step at 50°C, and further three washes at room temperature in 2 x SSC + 0.1% SDS.

The beads were blocked by incubation in 1% bovine serum albumin (BSA) in PBS-solution for 30 minutes at room temperature, followed by washing in PBS and resuspension in PBS containing 0.05% Tween-20 (PBST). The beads were then allowed to react with anti-biotin antibodies developed in goat (Sigma) for 30 minutes at room temperature. This was followed by four washes in PBST, and a second reaction to anti-goat antibodies conjugated to gold for 30 minutes at room temperature. After four washes in PBST and another four washes in sterile water, the beads were analysed under the microscope by electro-rotation as described in earlier examples.

As can be seen from Figure 17, the PCR product can be detected down to 0.5 pg/100 µg.

### Example 9

### Discrimination of Live and Dead Cryptosporidium Oocytes

Cryptosporidium is a parasite which infests the gut of grazing animals, often being found in untreated water, and upon occasion in drinking water.

We have used an electrorotation assay to discriminate between live and dead cryptosporidium oocytes. Plastics microspheres coated with anti-cryptosporidium antibody are mixed with a sample containing live and dead cryptosporidia. The formation of microparticle-organism complexes demonstrates the presence of the cryptosporidia. Subjecting the complexes to electrorotation at 100 Hz causes the dead organism complexes to rotate anti field whilst the live organism complexes do not. At 1 MHz, co-field rotation of both the live and the dead complexes is seen.

A similar approach may be applied to a wide range of other organisms, e.g. giardia, yeast, bacteria, virus infected cells, e.g. HIV infected leukocytes and leukaemia cells.

## Claims

1. A method of analysis by observation of field rotation properties characterised in that the method comprises forming a complex between a micro-particle, a linking moiety attached to the micro-particle and a target, which complex has field-rotation properties observably distinct from those of said micro-particle and attached linking moiety alone, and observing the said distinct field-rotation properties of the complex.

2. A method as claimed in Claim 1, wherein the field rotation properties observed are electro-rotation properties.

3. A method as claimed in Claim 1 or Claim 2, wherein the linking moiety is an antibody or an antibody fragment having antibody activity, an antigen, a nucleic acid probe or a nucleic acid analogue probe having selective affinity for complementary nucleic acid sequences, or avidin or an avidin like molecule.

4. A method as claimed in any preceding claim, wherein said complex further comprises a label bound to said target.

5. A method as claimed in Claim 4, wherein the label comprises a second linking moiety having an affinity for said target and a labelling moiety carried by said linking moiety.

6. A method as claimed in Claim 5, wherein said linking moiety is an antibody, an antibody fragment having antibody activity, an antigen, a nucleic acid probe, a nucleic acid analogue probe, avidin or an avidin like molecule.

7. A method as claimed in Claim 5 or Claim 6, wherein the labelling moiety carried by the linking moiety in the label is a micro-organism, a metal particle, a polymer bead or a magnetic particle.

8. A method as claimed in any one of Claims 4 to 7, wherein said label bound to said target comprises a magnetic particle.

9. A method as claimed in any preceding claim, wherein electro-rotation is observed using an electric field rotating at from 100 Hz to 1000 Hz.

10. A method as claimed in Claim 9, wherein the rotating electrical field has a strength of from 50 to 500 volts per centimetre.

11. A kit for use in analysis by electro-rotation comprising micro-particles bearing linking moieties adapted to form a complex with a particular target species and at least one of:-
(a) a sample receiving surface having thereon a pattern of electrodes surrounding an analysis field suitable for use in an electro-rotation assay; or
(b) an electro-rotation label adapted to form a complex with the same target species.

12. A kit as claimed in Claim 11, wherein the sample receiving surface is provided on a permeable member capable of filtering a sample to concentrate said target species.

13. A kit as claimed in Claim 12, comprising said sample receiving surface, wherein the electrodes are on the surface of a micro-porous filter through which a volume of sample is to be passed to trap micro-organisms on the membrane for binding to the micro-particle.

14. A kits as claimed in Claim 13, including means for passing a volume of liquid through said micro-porous filter providing said sample receiving surface.

## Patentansprüche

1. Verfahren der Analyse durch Beobachten von Feldrotations-Eigenschaften, dadurch gekennzeichnet, daß das Verfahren aufweist:
Bilden eines Komplexes zwischen einem Mikropartikel, einem mit dem Mikropartikel verbundenen verknüpfenden Molekülteil und einem Target, wobei der Komplex Feld-Rotationseigenschaften hat, welche sich deutlich von denjenigen des Mikropartikels und des verknüpfenden Molekülteils für sich allein unterscheiden, und Beobachten dieser charakteristischen Feldrotations-Eigenschaften des Komplexes.

2. Verfahren nach Anspruch 1, wobei die beobachteten Feldrotations-Eigenschaften Elektro-Rotations-Eigenschaften sind.

3. Verfahren nach Anspruch 1 oder 2, wobei das verknüpfende Molekülteil ein Antikörper oder ein Antikörperfragment mit Antikörper-Aktivität, ein Antigen, eine Nukleinsäure-Sonde oder eine Nukleinsäureanalog-Sonde mit selektiver Affinität für komplementäre Nukleinsäuresequenzen, oder Avidin oder ein avidinähnliches Molekül ist.

4. Verfahren nach einem der vorhergehenden Ansprüche, wobei der Komplex weiterhin eine mit dem Target verbundene Markierung aufweist.

5. Verfahren nach Anspruch 4, wobei die Markierung ein zweites verknüpfendes Molekülteil mit einer Affinität für das Target und ein markierendes Molekülteil, welches von dem verknüpfenden Molekülteil getragen wird, aufweist.

6. Verfahren nach Anspruch 5, wobei das verknüpfende Molekülteil ein Antikörper, ein Antikörperfragment mit Antikörper-Aktivität, ein Antigen, eine Nukleinsäure-Sonde, eine Nukleinsäureanalog-Sonde, Avidin oder ein avidinähnliches Molekül ist.

7. Verfahren nach Anspruch 5 oder 6, wobei das markierende Molekülteil, welches von dem verknüpfenden Molekülteil in der Markierung getragen wird, ein Mikroorganismus, ein Metallpartikel, ein Polymertropfen (Polymerperle) oder ein Magnetpartikel ist.

8. Verfahren nach einem der Ansprüche 4 bis 7, wobei die an das Target gebundene Markierung ein Magnetpartikel aufweist.

9. Verfahren nach einem der vorhergehenden Ansprüche, wobei Elektrorotation unter Verwendung eines elektrischen Feldes, welches bei zwischen 100 Hz und 1000 Hz rotiert, beobachtet wird.

10. Verfahren nach Anspruch 9, wobei das rotierende elektrische Feld eine Stärke von zwischen 50 und 500 Volt pro Zentimeter hat.

11. Kit zur Verwendung bei der Analyse durch Elektrorotation, das Mikropartikel, welche verknüpfende Molekülteile tragen, die geeignet sind, einen Komplex mit einer besonderen Targetart zu bilden, und mindestens eines der folgenden aufweist:
(a) eine eine Probe aufnehmende Oberfläche, auf welcher ein Muster von Elektroden ist, welche ein Analysefeld umgeben, welches für die Verwendung in einer Elektrorotations-Untersuchung geeignet ist; oder
(b) eine Elektrorotations-Markierung, welche geeignet ist, einen Komplex mit denselben Targetarten zu bilden.

12. Kit nach Anspruch 11, wobei die die Probe aufnehmende Oberfläche auf einem durchlässigen Element vorgesehen ist, welches eine Probe filtern kann, um die Targetart zu konzentrieren.

13. Kit nach Anspruch 12, mit der eine Probe aufnehmenden Oberfläche, wobei die Elektroden auf der Oberfläche eines mikroporösen Filters sind, durch welches ein Probevolumen hindurchgelangen soll, um Mikroorganismen auf der Membran zum Binden an dem Mikropartikel zu fangen.

14. Kit nach Anspruch 13, mit einer Einrichtung zum Durchleiten eines Flüssigkeitsvolumens durch den mikroporösen Filter, welcher die eine Probe aufnehmende Oberfläche bereitstellt.

## Revendications

1. Procédé d'analyse par l'observation des propriétés de rotation de champ, caractérisé en ce que le procédé comprend la formation d'un complexe entre une microparticule, une fraction de liaison fixée à la microparticule et une cible, ce complexe ayant des propriétés de rotation de champ visiblement distinctes de celles desdites microparticule et fraction de liaison fixée seules, et l'observation desdites propriétés distinctes de rotation de champ du complexe.

2. Procédé selon la revendication 1, dans lequel les propriétés de rotation de champ observées sont des propriétés d'électro-rotation.

3. Procédé selon la revendication 1 ou la revendication 2, dans lequel la fraction de liaison est un anticorps ou un fragment d'anticorps ayant une activité d'anticorps, un antigène, une sonde d'acide nucléique ou une sonde d'analogue d'acide nucléique ayant une affinité sélective pour des séquences d'acide nucléique complémentaires, ou l'avidine ou une molécule semblable à l'avidine.

4. Procédé selon l'une quelconque des revendications précédentes, dans lequel ledit complexe comprend en outre un marqueur lié à ladite cible.

5. Procédé selon la revendication 4, dans lequel le marqueur comprend une seconde fraction de liaison ayant de l'affinité pour ladite cible et une fraction de marqueur portée par ladite fraction de liaison.

6. Procédé selon la revendication 5, dans lequel ladite fraction de liaison est un anticorps, un fragment d'anticorps ayant une activité d'anticorps, un antigène, une sonde d'acide nucléique, une sonde d'analogue d'acide nucléique, l'avidine ou une molécule semblable à l'avidine.

7. Procédé selon la revendication 5 ou la revendication 6, dans lequel la fraction de marqueur portée par la fraction de liaison dans le marqueur est un micro-organisme, une particule métallique, une bille de polymère ou une particule magnétique.

8. Procédé selon l'une quelconque des revendications 4 à 7, dans lequel ledit marqueur lié à ladite cible comprend une particule magnétique.

9. Procédé selon l'une quelconque des revendications précédentes, dans lequel une électro-rotation est observée en utilisant un champ électrique tournant de 100 Hz à 1000 Hz.

10. Procédé selon la revendication 9, dans lequel le champ électrique tournant a une puissance de 50 à 500 V par centimètre.

11. Nécessaire pour l'utilisation dans une analyse par électro-rotation, comprenant des fractions de liaison portant des microparticules, adaptées pour former un complexe avec une espèce cible particulière et au moins l'un parmi :
(a) une surface recevant un échantillon sur laquelle se trouve un motif d'électrodes entourant un champ d'analyse approprié pour l'utilisation dans un essai d'électro-rotation ; ou
(b) un marqueur d'électro-rotation adapté pour former un complexe avec la même espèce cible.

12. Nécessaire selon la revendication 11, dans lequel la surface recevant l'échantillon est fournie sur un élément perméable apte à filtrer un échantillon pour concentrer ladite espèce cible.

13. Nécessaire selon la revendication 12, comprenant ladite surface recevant un échantillon, dans lequel les électrodes sont sur la surface d'un filtre microporeux à travers lequel un volume d'échantillon doit passer pour piéger des micro-organismes sur la membrane pour les lier aux microparticules.

14. Nécessaire selon la revendication 13, incluant des moyens pour faire passer un volume de liquide à travers ledit filtre microporeux fournissant ladite surface recevant un échantillon.
